(19) Europäisches Patentamt
European Patent Office
Office européen des brevets

(11) **EP 2 799 009 A1**

(12) **EUROPEAN PATENT APPLICATION**

(43) Date of publication:
05.11.2014 Bulletin 2014/45

(51) Int Cl.:
*A61B 5/11* (2006.01)  *A61B 5/00* (2006.01)

(21) Application number: 13165814.8

(22) Date of filing: 29.04.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Fraunhofer-Gesellschaft zur Förderung der angewandten Forschung e.V.
80686 München (DE)

(72) Inventors:
• Lötsch, Jörn
  60316 Frankfurt am Main (DE)
• Ultsch, Alfred
  35041 Marburg (DE)
• Oertel, Bruno
  60316 Frankfurt am Main (DE)
• Geisslinger, Gerd
  65812 Bad Soden (DE)

(74) Representative: Krauss, Jan
Boehmert & Boehmert
Anwaltspartnerschaft mbB
Patentanwälte Rechtsanwälte
Pettenkoferstrasse 20-22
80336 München (DE)

(54) **Non-invasive method for prediction of opioid-analgesia and opioid-blood-concentrations**

(57) The present invention pertains to a non-invasive method and apparatus for predicting or monitoring analgesia and blood levels of opioid drugs in a patient receiving pain treatment, e.g., during palliative treatment. The inventive method comprises the measurement of one or more, preferably two or more, surrogate markers of a patient. According to the present invention surrogate markers correlate with the level of analgesia in the opioid receiving subject and thus provide a non-invasive method to predict and monitor analgesia during a treatment. Even more, surrogate markers were identified which correlate with the blood-concentration of the opioid in the subject. Thus, the invention provides a valuable clinical tool to assess and control pain treatments with opioids. Disclosed is the prediction method, an apparatus suitable for performing the inventive methods as well as the apparatus for use in medical treatments, such as pain therapy.

Figure 1

**Description**

FIELD OF THE INVENTION

The present invention pertains to a non-invasive method and apparatus for predicting or monitoring analgesia and blood levels of opioid drugs in a patient receiving pain treatment, e.g., during palliative treatment. The inventive method comprises the measurement of one or more, preferably two or more, surrogate markers of a patient. According to the present invention surrogate markers correlate with the level of analgesia in the opioid receiving subject and thus provide a non-invasive method to predict and monitor analgesia during a treatment. Even more, surrogate markers were identified which correlate with the blood-concentration of the opioid in the subject. Thus, the invention provides a valuable clinical tool to assess, monitor and control pain treatments with opioids. Disclosed is the prediction method, an apparatus suitable for performing the inventive methods as well as the apparatus for use in medical treatments, such as pain therapy.

DESCRIPTION

The present invention is directed to valuable tools for use in pain treatment (analgesia) in larger mammals, in particular in humans. Opioid analgesics such as e.g. morphine and its derivatives are the most powerful analgesic drugs. The pain relieving activity of opioid analgesics includes a depressive effect on the central nervous system with respiratory depression being one that sometimes even ends fatal. Opioid analgesics are invaluable for the treatment of severe acute or chronic pain as, for example, may occur in bone degenerative diseases and cancer conditions. They are easy to administer and they provide effective pain relief in most patients. Due to the lack of acute organ toxicity the doses of morphine and other strong opioids can be increased to relatively high levels. Yet, dosing of opioids to achieve optimal analgesia in a patient is still not satisfactory controlled.

A well-known and widely used opioid is remifentanil, which is marketed as the hydrochloric salt from Glaxo Wellcome Inc. under the tradename ULTIVA® (remifentanil hydrochloric salt). It is a mu-opioid agonist indicated for four administration as (1) an analgesic agent for use during the induction and maintenance of general anesthesia for in-patient and out-patient procedures, (2) for continuation as an analgesic into the immediate postoperative period under the direct supervision of an anesthesia practitioner in a postoperative anesthesia care unit or intensive care setting, and (3) as an analgesic component of monitored anesthesia care. Remifentanil is chemically designated as a 3-[4-methoxycarbonyl-4-[(1-oxopropyl)phenylamino]-1-piperidine]propanoic acid methyl ester, hydrochloric salt, $C_{20}H_{28}N_2O_5 \cdot HCl$, with a molecular weight of 412.91. Because of the rapid onset of action of remifentanil, accurate dosing and monitored care are important in order to avoid adverse effects. This is not always suitable for outpatient procedures performed by general practitioners or by patient controlled infusions.

For example postoperative patients, or chronic patients suffering from severe diseases such as cancer, generally suffer a great deal of pain as a result of the surgical procedure, or the nature of the disease. The physician usually prescribes strong analgesics or painkillers in the form of pills or tablets to relieve this symptom for most patients. For some cases, analgesics are prescribed which can be self-administered by the patient through a "patient-controlled analgesia" (PCA) device or pump. The typical PCA device is programmed to deliver a predetermined amount of opioid (according prescription, or drug leaflet), such as morphine, through a connected intravenous tube whenever the patient presses a button according to the patient's perceived needs. While the PCA device relieves pressure on nursing resources and grants superior pain control, the patient could easily induce an accidental overdose if the administration of the analgesic is left unchecked or if the patient is hypersensitive to the analgesic. Further possibilities for PCAs are patient-controlled epidural analgesia, patient-controlled regional analgesia or transdermal systems (patches).

However, draw-backs in PCA supported pain therapy occur in the case of mentally impaired patients, patients with severe physical damages (problems with handling of the devices) as well as young and very old patients.

Another option in analgesia is a system that infuses the drug based on the calculations of a computer program, known as "target controlled infusion" (TCI). Empirically determined models based on population kinetics together with data of the pharmacokinetic and-dynamic profile of a given medicament and patient specifics provide the basis for a calculation of the drug administration in order to achieve optimal opioid concentrations at the target area (central nervous system). This system though does not allow the patient to individually adjust drug dosage, and therefore often does not meet the patient's individual needs. Further, the data on pharmacokinetics and -dynamics vary significantly within and in between individual patients. Thus TCI system often cannot provide appropriate analgesia.

Surrogate parameters are important tools in drug development and monitoring. They provide easy and quick access to information about parameters of interest, which can be measured directly only with difficulty. Miotic opioid effects have recently been proposed as a suitable non-invasive surrogate for opioid blood concentrations (Kharasch et al., 2003) to replace invasive sampling and to provide information about blood concentrations quicker than after analytical assays.

However, apart from pharmacokinetic studies where analytical assays should not be replaced by a surrogate, and in a forensic context, when opioid dosing is in question without available blood samples (Lötsch et al., 2006), opioid blood concentrations mainly serve as an auxiliary measure for the subsequently linked clinical opioid effects. More important are surrogates with relevance for clinical effects as inadequate analgesia due to opioid under-treatment is still a vital topic in modem medicine (Sinatra, 2010, Moskovitz et al., 2011, Wu and Raja, 2011, Simone et al., 2012). An effect-compartment controlled target-controlled infusion system, like recently proposed for anesthesia (Sahinovic et al., 2010), constantly fed with information from a surrogate marker for opioid analgesia, could represent one of the potential strategies to optimize opioid-based pain therapy. However, whether miotic opioid effects are suited to draw conclusions about clinically relevant opioid effects is uncertain.

An opioid that would particularly benefit from a relevant surrogate is remifentanil because concentration assessment requires arterial sampling (Egan et al., 1993, Egan et al., 1996, Minto et al., 1997a, Egan et al., 2004, Noh et al., 2006, Oertel et al., 2012b), associated with the risk of arterial occlusion (Evans and Kerr, 1975, Bright et al., 1993, Oertel et al., 2012b), a challenging sample handling is needed (Lötsch and Angst, 2003) and analytical methods are established in only a few specialized laboratories of the world.

In view of the above described major drawbacks in the background art, it is the objective of the present invention to provide novel clinical tools that allow the medical practitioner to determine the level of analgesia and the level of side effects in a patient receiving pain treatment with one or more opioids, as well as an alternative for measuring opioid blood-concentrations without arterial sampling.

The above problem is solved in a first aspect by a method for the non-invasive prediction or monitoring of analgesia, and/or opioid blood-concentrations, in a subject which received at least one opioid dose, comprising acquiring at least one surrogate marker of said subject before, and at the same time or at least once after, the opioid administration; and determining therefrom said analgesia, or opioid blood-concentration, in said subject. In a particularly preferred embodiment at least two, three or more surrogate markers are acquired of said patient. In this setting said analgesia, or opioid blood-concentration, is determined by linear combination of said two, three or more surrogate markers.

In context of the herein described invention it was surprisingly discovered that the linear combination of more than one surrogate marker (parameter) allows for an adequate and statistically stable prediction of the level of analgesia in a subject receiving opioids. Furthermore, surrogate markers such as the pupil light reflex provided at least in the case of the opioid remifentanil correlation with the blood-concentration of the opioid, and thus renders arterial sampling superfluous.

Pharmacodynamic surrogate parameters combined by means of a mathematical equation provide a novel tool for the prediction of relevant clinical drug effects that is superior even to the prediction of the effects by measured drug concentrations. The here presented combined surrogate approach seems a suitable path to be followed during early-phase drug development strategies or implanted in therapeutic drug monitoring and controlled dosing systems.

In the context of the present invention the terms "biomarker" and "surrogate marker" shall be understood to pertain to two different parameters. Biomarkers are defined as a laboratory measurement that reflects the activity of a disease process. Typical biomarkers include blood levels of proteins or other molecules, etc., which in essentially all cases, are markers that quantitatively correlate (either directly or inversely) with disease progression and constitute clinical end points. A "surrogate marker" on the other hand must be understood as a substitute for a clinical endpoint, without necessarily having a real relationship with the clinical end point. Because the surrogate markers are employed as a percent change in status, virtually any indicator can be used. Surrogate markers could include any measure of the effectiveness of an opioid's action. Given the effectiveness of the opioid's action, relative to the surrogate markers, the prediction of analgesia or concentration may be used in the clinic to determine a change in drug dose of the subject. Conversely, by employing this system, one could determine the expected result of a drug dose change on the surrogate markers.

In preferred embodiments of the present invention the surrogate marker is selected from the group consisting of pupil size, pupil reaction to light stimuli, tracking test performance, respiratory rate, arterial oxygenation, partial pressure of transcutaneous carbon dioxide, EEG derived parameters, clinical parameters of tiredness, nausea, drowsiness, vertigo, and itching. Particularly preferred are the markers: pupil size, pupil reaction to light stimuli, tracking test performance.

The surrogate markers pupil size and pupil reaction (constriction or dilation) to light stimuli are usually tested in preferred embodiments of the invention via a pupilograph or pupilometer. Usually a pupilometer is a hand-held portable electronic instrument that assesses and records pupil size and reaction to stimulus.

The surrogate marker tracking test performance, or compensatory tracking task, is a task that assesses eye-hand coordination, in which the subject is operating a display that has an indicator and a zero point using a joystick, computer mouse, trackball, or other controlling device. The user must try to keep the indicator within the zero point while the indicator is being acted upon by outside forces. Such tracking tasks can be of varying design, which shall not be restrictive for the embodiments of the present invention.

Surrogate markers such as respiratory rate, arterial oxygenation, partial pressure of transcutaneous carbon dioxide, EEG derived parameters are all well known to the clinical practitioner and can be acquired by standard means known in the art. Surrogate markers corresponding to clinical parameters of tiredness, nausea, drowsiness, vertigo, and itching, may be, for example, acquired by means of paper-administered visual analog scales, e.g. from zero to 100.

One further embodiment of the invention pertains to a method as described herein above wherein said blood opioid concentration is determined according to equation I:

$$(I) \quad C = Effect \cdot EC_{50}/(E_{max} - Effect),$$

wherein
C is the predicted blood-concentration of said opioid,
*Effect* is the relative change of said surrogate marker from the baseline
$EC_{50}$ is the concentration of said opioid at half maximum effect
$E_{max}$ is the maximum effect.

In an even more preferred embodiment the surrogate marker light reflex amplitude (pupil reaction to light) is used in the above equation I which was found in the context of the herein described invention to provide excellent predictions for the blood remifentanil concentrations. This is particularly useful, because the blood concentrations of remifentanil, as mentioned above, must be to date determined by arterial sampling - which is due to various risks still unfavorable. In the case of remifentanil, in the above equation I said *Effect* is provided in percent change from baseline, and/or said $EC_{50}$ for remifentanil is 0.6 ng/ml, and/or said $E_{max}$ is -100%.

Yet, it was furthermore discovered that the linear combination of more than one surrogate marker allowed for an even better prediction of opioid blood concentrations. Thus, it is more preferred that in the herein disclosed methods more than one surrogate marker, preferably two or three, are combined with each other in a linear equation for a given opioid, which than allows for the prediction of the blood concentrations of said opioid in a subject.

Preferably in the embodiment of the linear combination of more than one, preferably two or three, or more surrogate markers, the opioid blood-concentration is determined by equation (II)

$$(II): \quad C_{predicted} = k \cdot Effect_{\text{Pupil diameter}} - m \cdot Effect_{\text{Light reflex amplitude}} - n \cdot Effect_{\text{Tracking performance}} + w,$$

wherein k, m, n and w, are coefficients ranging from $-\infty$ to $\infty$, depending on the scaling.

The above coefficients may vary depending on the opioid for which a prediction of monitoring of blood-concentrations is needed. For remifentanil, k is 0.10316, m is 0.095613, n is 0.060719 and w is 0.038027. Of course, the above equation may include the *Effects* of other surrogate markers to increase accuracy, or one or more surrogate marker is substituted with a different surrogate marker. Also as mentioned above, the linear combination may only include the linear combination of two surrogate markers, or on the other hand more than three surrogate markers.

Preferably the above method is for predicting analgesia in a subject receiving opioid treatment. Thus it is preferred that said analgesia is predicted by linear combination of more than one surrogate marker, preferably two or three, according to equation (III)

$$(III): \quad Analgesia_{predicted} = q \cdot Effect_{\text{Pupil diameter}} - s \cdot Effect_{\text{Light reflex amplitude}} + t \cdot Effect_{\text{Tracking performance}} + v,$$

wherein
*Effect* is the relative change of said surrogate marker from the baseline (percent from baseline), and
q, s, t, v are coefficients ranging from $-\infty$ to $\infty$, depending on the scaling.

The above coefficients may vary depending on the opioid for which a prediction of monitoring of analgesia is needed. For remifentanil, q is 4.7547, s is 2.4799, t is 0.45914 and v is 47.7756. Of course, the above equation may include the *Effects* of other surrogate markers to increase accuracy, or one or more surrogate marker is substituted with a different surrogate marker. Also as mentioned above, the linear combination may only include the linear combination of two surrogate markers, or on the other hand more than three surrogate markers.

The finding of the present invention suggests that predictions of opioid analgesia from suitable pharmacodynamic surrogates are superior to that from opioid blood concentrations. Even under apparent steady state conditions at effect site, the transfer between blood and CNS is subject to interindividual variance. In addition, the resulting opioid effects depend on local concentrations and on the local opioid receptor density and signaling efficiency that is known to vary among pain-relevant brain regions. These differences may be better captured with pharmacodynamic surrogate parameters according to the method of the present invention than by associating effects solely to concentrations.

Opioids are among the oldest drugs in existence, and remain a mainstay of pain management. The term "opioid" shall refer to the following substances. Opium, the original opioid, is derived from poppy plants. "Opiates" are natural derivatives of opium, and include morphine, methadone, and heroin. "Opioids" are a broader class of drugs, that includes opium, opiates, and synthetic drugs with the same pharmacological effect of opium. Commonly used, and herein preferred synthetic opioids include meperidine, fentanyl, alfentanil, sufentanil, and remifentanil. Particularly preferred is remifentanil.

In another aspect of the invention there is provided a method for treating, monitoring or controlling analgesia in a patient receiving an opioid treatment, comprising the steps of predicting the analgesia according to the prediction method of the herein above described invention, and calculating based on the predicted analgesia a revised dose of said opioid. Said revised dose shall provide a better analgesia, which means that the analgesia shall meet the individual needs of the actual patient, for example a higher level of analgesia in case of insufficient pain suppression, or a lower level on analgesia in case of unnecessary strong , or even dangerous opioid effects in said patient, for example in order to avoid respiratory depression in the patient.

In this aspect it is preferred that the surrogate markers are constantly observed and updated while the opioid is administered to the patient. In this way the level of analgesia can be controlled and monitored in order to adjust drug levels to the individual needs of the patient, or to avoid dangerous overtreatment and to enable the timely initiation of counter treatments. By this way it will be possible for clinical practitioners to improve pain treatments that to date suffer from under-treatment due to cautious medical personal.

The problem of the invention is furthermore solved by an apparatus or system for performing the inventive method in all of the embodiments described herein. Thus in certain embodiments of the invention preferred is an apparatus or system for the prediction of analgesia, or opioid blood-concentrations. The apparatus may comprise

    a. Means for acquiring said at least one surrogate marker of a subject, and/or means for accepting as input said at least one surrogate marker,
    b. Means for storing the recorded values of said at least one surrogate markers,
    c. Means for calculating or monitoring said predicted analgesia, or opioid blood-concentrations, and
    d. Optionally, means for display/output of the calculated analgesia, or opioid blood-concentrations.

It is preferred that the apparatus or system according to the invention comprises a. means for acquiring at least two or three, or more, surrogate markers of a subject, and/or means for accepting as input at least two, three, or more, surrogate markers.

This apparatus or system includes as a means for calculating or monitoring a general purpose computer, comprising an input means, which is preferably a keyboard and/or a mouse and/or trackball and/or a joystick, a display/output means, preferably a video display screen, a data storage means, preferably a hard disk drive, and a processor. It is preferred that on this computer the computer program receives input data from a physician or the patient regarding the current values of the individual surrogate markers as the percent response of the patient based on the surrogate markers used to monitor the opioid. Additional input may be the current drug dosage, and the initial drug dosage the patient received. Further additional input may include individual patient characteristics such as gender, age, weight, etc.

It is preferred that within the apparatus embodiment of the invention said predicted analgesia, or opioid blood-concentrations, is calculated by any of the above equations (I) to (III), or in case other combinations of surrogate markers are use, the respective equations adjusted to said surrogate markers, or marker combinations.

In one further embodiment the apparatus according to the invention further comprises means for applying a dose of said opioid to a subject. Such means may be for example an electronically controlled infusion system, a syringe or the like. Such automated systems for the application of drugs are well known within the art.

The present invention supplies a clinical tool to evaluate the analgesia or opioid blood concentrations in a patient in a non-invasive manner. Even more, the invention for the first time described that the use of surrogate markers provides an even better estimation of analgesia than the measuring of blood concentrations. Thus, the apparatus of the invention in one additional preferred embodiment is characterized in that the amount and/or infusion rate of said dose of said opioid is calculated based on the predicted analgesia, and/or predicted opioid blood-concentration.

The apparatus may further comprise safety measures known in the art. Preferred are means for applying to a subject a countermeasure to the opioid, for example an infusion of naloxone. Additionally or alternatively the apparatus may comprise means for acquiring respiratory depression in said subject, and/or means for accepting as input the parameter for respiratory depression of said subject. If respiratory depression occurs, safety measures can be initiated in time in order to prevent the subject to suffer any harm from the opioid infusion.

The apparatus may furthermore comprise means for accepting as input the subject's initial and/or current opioid dose.

The invention additionally solves the above problem by the above described apparatus for use in medicine, preferably for use in the pain treatment of a patient, such as a pain treatment involving the opioid remifentanil.

The present invention will now be further described in the following examples with reference to the accompanying figures and sequences, nevertheless, without being limited thereto. For the purposes of the present invention, all references as cited herein are incorporated by reference in their entireties. In the Figures:

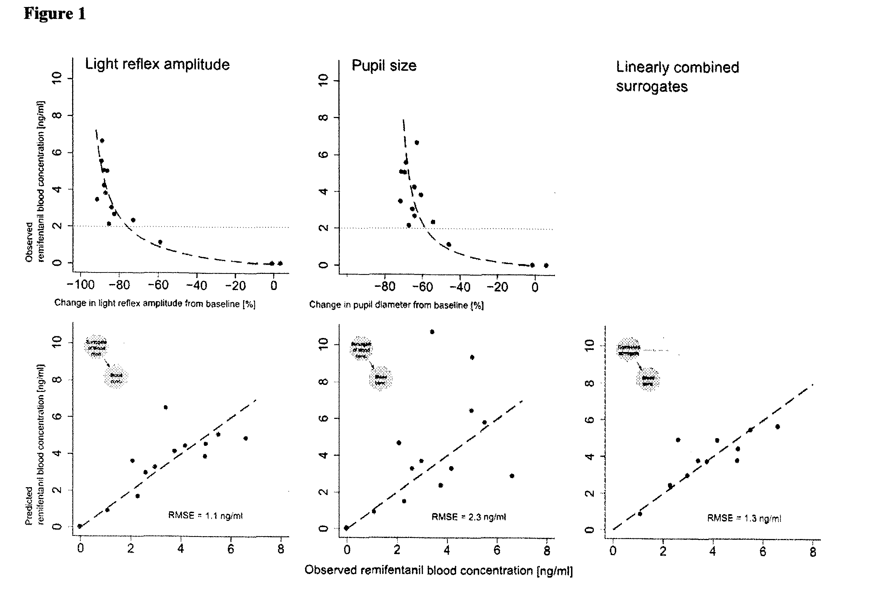

Figure 1: : Association of remifentanil blood concentrations (n = 14) with pupil derived parameters (Left: light reflex amplitude, right: pupil diameter). The top panels show the sigmoidal relationship between blood concentrations and pupil measures $C = \frac{Effect \cdot EC_{50}}{E_{max} - Effect}$, dashed curbed lines, with values of $E_{max}$ = -100% and $EC_{50}$ = 0.6 ng/ml for light reflex amplitudes, and of $E_{max}$ = -75.1 % and $EC_{50}$ = 0.6 ng/ml for pupil diameters), the bottom panels display the concentrations predicted (ordinate) using the sigmoidal relationship plotted versus the observed concentrations (abscissa). The line of agreement between predictions and observations is given as a dashed diagonal; dots above this line show over-predictions. A linear surrogate combination was also suitable to predict blood concentrations: $C_{predicted}$=0.10316 · $Effect_{Pupil}$ diameter - 0.095613 · $Effect_{Light}$ reflex amplitude - 0.060719 · $Effect_{Tracking}$ performance + 0.038027 ($3^{rd}$ bottom panel from left), only slightly less accurate than the predictions obtained with the light reflex amplitude using a sigmoidal relationship (n = 12; zero concentrations excluded). *RMSE:* root mean square error between predicted and observed values.

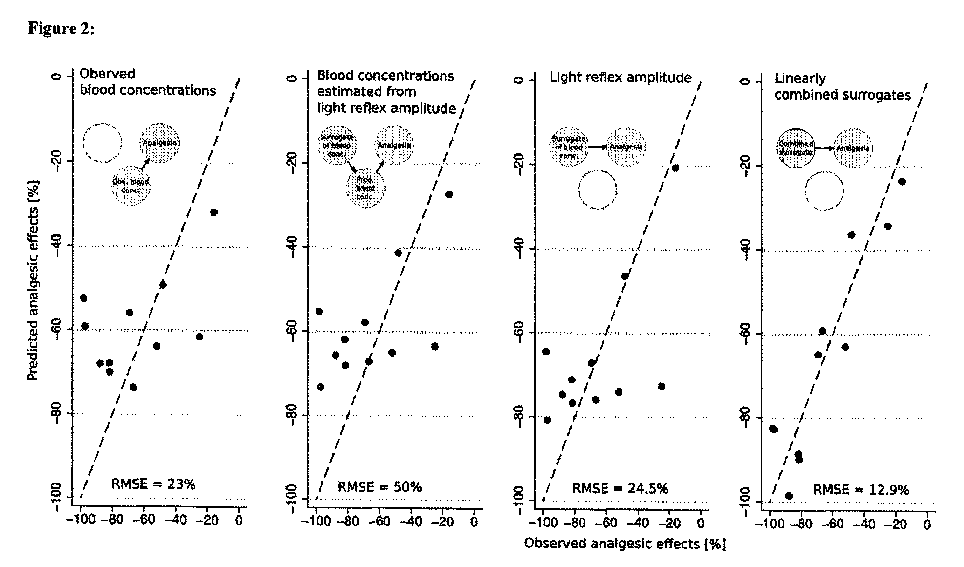

Figure 2: Predictions of analgesic effects (n = 11; outliers excluded) from observed remifentanil blood concentrations and different surrogate parameters as given at the top of each panel, with indirect or direct prediction paths indicated below. Left panel: Prediction of analgesia from measured remifentanil blood concentrations would be possible with an RMSE of 23% (*Analgesia$_{Obs}$* = 10.34 · $C_{Remifentanil}$ + 20.53). When using the effects on the light reflex amplitude to directly predict the analgesic effects (*Analgesiap$_{Pred}$* = 1.84,1 · $Effect_{light\ reflex}$ + 86.9372), the prediction improved to a level that was comparable to that obtained from observed remifentanil blood concentrations ($3^{nd}$ panel from left). Only when using a linear combination of surrogate parameters (e.g. *Analgesia$_{predicted}$* =4.7547 · $Effect_{Pupil\ diameter}$ - 2.4799 · $Effect_{Light\ reflex\ amplitude}$ + 0.45914 · $Effect_{Tracking\ performance}$ + 47.7756), the precision became acceptable (RMSE = 12.9%) and was superior to that obtained from observed remifentanil blood concentrations. A linear surrogate combination was also suitable to predict blood concentrations (compare Figure 1). The lines of agreement between predictions and observations are given as dashed diagonals; dots above these lines show over-predictions.

EXAMPLES

**Materials and Methods**

*Subjects*

Sixteen volunteers (aged 19 - 36 years, all within ± 10 % of their normal body weight, eight men) participated in this double-blind, randomized, single-occasion study. Subjects were determined to be healthy by examining the medical history, present physical condition and routine clinical laboratory tests and abstained from prescription/over-the-counter medications, alcohol and food for 30 days, 24 hours and six hours before the experiments, respectively. Effect measures and clinical effects were assessed in the order: visual acuity (duration: 5 min), clinical parameters (1 min), pupil sizes and reactions to light stimulation (5 min), analgesic effects and concomitant assessment of the tracking performance (20 min). Additional data from this study, non-redundant with the present analysis, has been published in another context in (Lötsch et al., 2001) and (Oertel et al., 2012b).

*Medication*

Remifentanil (Ultiva®, courtesy of Glaxo Wellcome GmbH & Co., Hamburg, Germany) is an ultra-fast acting opioid which, due to its ester structure, is rapidly degraded in the blood by non-specific tissue and plasma esterases (Egan. 1995) rendering venous blood concentrations irrelevant (Hermann et al., 1999) and causing the necessity of arterial blood sampling (Oertel et al., 2012b). Due to its lipophilicity, remifentanil is quickly transferred between blood and central nervous effect sites with a first-order equilibration half-life of 1 - 2 min (Egan et al., 1996). To circumvent this delay, steady-state concentrations were used. Remifentanil was administered intravenously by means of computerized infusion using STANPUMP ((Shafer et al., 1990) freely available from Steven L. Shafer, MD at http://www.opentci.org/lib/exe/fetch.php?media=code:stanpump.zip, accessed October 13, 2012) with published population pharmacokinetic parameters (Minto et al., 1997a, Minto et al., 1997b). Different target concentrations at the effect site were studied (0, 1.2, 2.4, 3.6, 4.8, and 6 ng/ml as used previously (Lötsch and Angst, 2003) and for better resolution in the lower concentration range additionally 1.8 and 3 ng/ml). One hour equilibration time was allowed to ensure steady state, which also allowed the subjects to adapt to the opioid effects and experimental conditions and was considerably longer than suggested by simulations of steady state conditions, which, however, were based on population mean values without including intersubject variability. Therefore, the blood concentrations could be taken as a substitute of the effects site concentrations. To reduce opioid exposure, one target concentration was assigned to one man and woman; a simplified design adapted from "single ascending dose studies" where only a few subjects receive a particular drug dose while another dose is administered to a new group of subjects, as often employed in drug safety studies to limit the number of subjects exposed to a dose and minimize the efforts when many doses are tested (Bouman-Thio et al., 2008, Jiang et al., 2009, Lin et al., 2010, Padhi et al., 2011).

***Measurements***

*Remifentanil blood concentrations*

Blood (4 ml each) was sampled into lithium-heparin tubes at 0, 0.33, 0.5, 0.75, 1, 1.25, 1.5, 2, 2.5 and 3 h relative to the start of infusion (detailed concentrations given elsewhere (Oertel et al., 2012b)). As described previously (Oertel et al., 2012b), tubes were gently shaken to mix blood and anticoagulant. Immediately, two aliquots of exactly 1 ml were pipetted into plastic storage tubes containing precisely 20 $\mu$l citric acid (50 % w/w) for exact pH control peremptory to prevent remifentanil hydrolysis (Selinger et al., 1994). Manipulations were made with tubes on ice which were subsequently stored at -80°C. Remifentanil concentrations were measured using a validated LC-MS/MS assay (Jasco LC-unit, Groß-Umstadt, Germany, Sciex API 3000 tandem mass spectrometer, Langen, Germany). Mass transitions used for quantitation and qualification were m/z $377 \rightarrow 113.1$ (collision energy 41 eV) and m/z $377 \rightarrow 81.0$ (collision energy 59 eV), respectively. Standards were analyzed at concentrations of 0.01 - 10 ng/ml (internal standard alfentanil). Quality control samples were randomly analyzed. The interday precision of the assay was 8% with accuracy within 10% of nominal values.

*Candidate surrogate effect measures*

**Pupil sizes** and reactions to light stimulation were tested using a pupilograph (CIP, Amtech GmbH, Weinheim, Germany). The **pupil reaction to light stimuli** of 9766 Candela/m$^2$ (wave length 585 nm, stimulus duration 0.2 s) was sampled at a frequency of 250 Hz for 2 s with implemented CCD-line scan camera. Each measurement was repeated five times, and a total of three measurements at baseline and three during opioid infusion were done before averaging the data. The light in the windowless laboratory was kept at 13.6 Lux and was reduced to 0.3 Lux 3 min before measurements.

During pain tests (see below) but limited to the periods between pain stimuli, subjects executed a **tracking task** on a computer screen (idea/programming: Kobal, Müller and Hummel (Hummel and Kobal, 2001)). Using a joystick, they had to keep a small red square inside a larger green one that randomly moved across the screen at eye-level at a distance of approximately 2 m from the subject's eyes. The tracking performance was determined by measuring how long the subjects lost contact with the independently moving square (0 to 100 % successful performance in tracking (Hummel et al., 1994)).

*Effects of clinical interest*

**Analgesic effects** were assessed using an experimental pain model that employed nociceptive stimuli consisting of short pulses (200 ms, 75 % v/v, n = 15) of gaseous carbon dioxide ($CO_2$) applied to the right nasal mucosa (Kobal, 1985) at an interstimulus interval of 30 s. The $CO_2$ stimuli were applied via a thin Teflon tube (outer diameter of 0.5 mm), embedded into a constantly flowing stream of compartment air (8 1/min) with controlled temperature (36.5 °C) and humidity (80 % relative humidity). After each stimulus, the subjects rated its intensity via a visual analog scale displayed on a computer monitor, ranging from zero ("no pain") to 100 ("severe pain"). For statistical evaluation, the estimates of individual subjects were averaged. Subjects were rated selected clinical parameters such as **tiredness, drowsiness and nausea** as typical acute opioid effects by means of paper-administered visual analog scales (100 mm length, ranging from zero, "no such symptom", to 100, "symptom experienced at maximum intensity").

**Visual acuity** was assessed by displaying a standard chart of 10 lines of letters at decreasing sizes for reading at a distance of 6 m from the participant. An illumination of 700 Lux on the chart and of 450 adjacent to the chart was observed (Buch et al., 2001). The visual acuity was calculated from the number of the line with the smallest letters that were correctly read.

*Data analysis*

Data acquired during the remifentanil infusion was transformed to percent changes from baseline. Subsequent exploration for correlations was done by calculating Spearman's correlation coefficient (Spearman, 1987). This measures the similarity of the orderings of ranked data, thereby taking into account that some of the tested relationships were probably non-linear. For the latter reason, a sigmoid pharmacodynamic model (Holford and Sheiner, 1982),

$$Effect = \frac{E_{max} C^{\gamma}}{EC_{50}^{\gamma} + C^{\gamma}},$$ was subsequently fitted to the data, with $E_{max}$ and $EC_{50}$ denoting the maximum effect and the concentration at half-maximum effect as a measure of drug potency, respectively. The shape factor, $\gamma$, was fixed at a value of 1 as the data did not allow to estimate a shape factor different than 1 as indicated by 95% confidence intervals of $\gamma$ always including the value of 1 and the non-significant outcomes of F-tests comparing the simpler (i.e., $\gamma = 1$) with the more complicated (i.e., $\gamma$ allowed to differ from a value of 1) models. As one measurement per subject was available, this was a pooled analysis, not providing separate estimates of intraindividual and interindividual variabilities. Nevertheless, the data was approximately log-normally distributed, which justifies the use of sigmoid pharmacodynamic models (Holford and Sheiner, 1982).

Subsequently, predictions of blood concentrations by candidate surrogate parameters were assessed using the pharmacodynamic model rearranged for C, i.e., $C = \frac{Effect \cdot EC_{50}}{E_{max} - Effect}$ (inverse sigmoidal pharmacodynamic model).

Alternatively, linear predictions were explored using Matlab's (MathWorks, Natic, MA, USA) robust linear regression estimation (DuMouchel and O'Brien, 1989). The prediction performances were comparatively judged on the basis of the root mean square error between predicted, P, and observed, *O*, values, calculated as $RMSE = \sqrt{\frac{1}{n} \sum_{i=1}^{n} (O - P)^2}$,

measured in the same units as the data and representative of the standard error of the predictions. The RMSE is the square root of the squared deviations from the model to the predictions. It represents an absolute measure of goodness-of-fit whereas the R-squared measure is a relative measure of fit (Hosmer et al., 1991). Lower values of RMSE indicate a better fit. As RMSE is a sum, it is susceptible to outliers. Therefore, RMSE should always be compared with a visualization of the data (e.g. Figure 2 where the lowest RMSE, 12.9, indeed indicates that the particular prediction model is the best).

Direct prediction of clinical effects by surrogate parameters was also analyzed. Subgroups of data were identified by means of hierarchical clustering. As surrogate effects are related by an inverse sigmoidal pharmacodynamic model to the concentrations, which are related to effects by a sigmoidal model, surrogate and clinical effects can be regarded as linearly related and analyzed by means of (multiple) linear regression.

**Example I: Prediction of remifentanil blood concentrations from surrogates**

Data was available from 14 subjects as of three dropouts (at target concentrations of 1.8, 2.4 and 6 ng/ml) only one was replaced (1.8 ng/ml). Two subjects dropped out at an early stage of the experiment, one because of anxiety shortly after the remifentanil infusion was started and another one due to excessive vomiting and nausea. A third dropout was due to bradycardia that caused the medical decision to stop the remifentanil infusion.

**Table 1:** Non-parametric Spearman rank correlation matrix among opioid effect measures (n=14; percent changes in effect measures from baseline served as the basis for this non-parametric correlation analysis). The correlation coefficient p is given with the respective p-values.

| Changes in | Rank correlation matrix | | | | | | | |
|---|---|---|---|---|---|---|---|---|
| | Blood concentrations | Pain | Pupil diameter | Light reflex | Visual acuity | Tiredness | Drowsiness | Nausea |
| Pain | -.565* p=0.035 | | | | | | | |
| Pupil diameter | -.629 p=0.016 | .644 p=0.013 | | | | | | |
| Light reflex | -.862 p<0.001 | .521 p=0.056 | .776 p=0.001 | | | | | |
| Visual acuity | -.454 p=0.103 | .334 p=0.244 | .745 p=0.002 | .712 p=0.004 | | | | |
| Tiredness | .405 p=0.151 | -.486 p=0.078 | -.609 p=0.021 | -.640 p=0.014 | -.718 p=0.004 | | | |
| Drowsiness | .533 p=0.050 | -.245 p=0.398 | -.594 p=0.025 | -.514 p=0.060 | -.639 p=0.014 | .545 p=0.044 | | |
| Nausea | .558 p=0.038 | -.287 p=0.319 | -.667 p=0.009 | -.632 p=0.015 | -.766 p=0.001 | .316 p=0.271 | .562 p=0.037 | |
| Tracking | -.801 p=0.001 | .692 p=0.009 | .692 p=0.009 | .742 p=0.004 | .689 p=0.009 | -.698 p=0.008 | -.696 p=0.008 | -.650 p=0.016 |
| *: From this significant non-parametric rank correlation does not follow that pain could be predicted from plasma concentrations using a linear relationship between the arterial remifentanil concentrations and the changes in pain. Indeed, such a relationship of $Analgesia_{Obs} = 10.34 \cdot C_{Remifentanil} + 20.53)$ predicted pain with an RMSE of 28.4%, which was worse than the using the sigmoid pharmacodynamic model (RMSE 23%, see also Figure 2). | | | | | | | | |

Median$_{1-3h}$ arterial blood concentrations of remifentanil ranged between 1.1 and 6.6 ng/ml (plus two zero concentrations). Recorded subjective ratings of the clinical parameters tiredness (0 - 66 mm VAS), nausea (0 - 12 mm VAS) and drowsiness (0 - 5 mm VAS) raised fowling the administration of remifentanil to 28 -100, 0 -23, and 0 - 100 mm VAS, respectively, with higher symptoms associated with high opioid concentrations (further details not shown). The correlation matrix (Table 1) further showed that remifentanil effects on the light reflex amplitude were highest (negative) correlated with its concentrations, followed by its effects on the tracking performance and on pupil diameters.

Considering the proposal of miotic opioid effects as a noninvasive surrogate for its conventional pharmacokinetics (Kharasch et al., 2003), the first part of the analysis comparatively addressed the prediction of remifentanil blood concentration by candidate surrogate parameters. The best prediction of remifentanil blood concentrations (RMSE = 1.1 ng/ml) was obtained with the light reflex amplitude (Figure 1) associated sigmoid-shaped to the concentrations ($EC_{50}$ = 0.6 ng/ml, $E_{max}$ = -100 %; these parameters had been obtained by a standard fit of pooled data using a classical sigmoid pharmacodynamic model. Details about goodness-of-fit are: the standard error of estimate of EC$_{50}$ = 0.1, E$_{max}$ did not significantly differ from a value of -100 % and had therefore been fixed, and goodness-of-fit: R$^2$ = 0.984). Pupil diameters (Kharasch et al., 2003) and the tracking performance produced comparatively inferior predictions (RMSE = 2.6 ng/ml and 3.2 ng/ml, respectively).

**Example II: Prediction of analgesia from surrogates**

In the second part of the analysis, the utility of the surrogates to predict more clinically relevant remifentanil effects, in particular analgesia, was analyzed. Two subjects with zero concentrations and a subject with hyperalgesia (increased pain ratings by 23 % from 25 to 30.7 mm VAS during steady-state blood concentrations of 2.1 ng/ml, target 1.8 ng/ml) formed separate clusters and were excluded. Predicting the analgesic effects on the basis of the observed remifentanil blood concentrations ($E_{max}$ = -100 % and $EC_{50}$ = 2.4 ng/ml; again, these values had been obtained using a standard sigmoidal model: the standard error of the estimate of $EC_{50}$ was 4, $E_{max}$ did not significantly differ from a value of -100 % and had therefore been fixed, and R$^2$ was 0.274) provided an RMSE of 23 %. Replacing in $E_{max}$ model the observed concentrations with those predicted on the basis of the pupil light reflex amplitude resulted in an RMSE of 50 % (Figure 2). In contrast, the light reflex amplitude provided a prediction of analgesia with an RMSE = 24.4 % and pupil sizes of 17.8 %.

However, the prediction of analgesia (Figure 2) substantially improved when using a linear combination of all three surrogate parameters as

$$Effect_{Analgesia} = 4.7547 \cdot Effect_{Pupil\ diameter} - 2.4799 \cdot Effect_{Light\ reflex\ amplitude} + 0.45914 \cdot Effect_{Tracking\ performance} + 47.7756$$

This Model gave a good RMSE of 12.9 % (Figure 2). Similar linear combinations of surrogate parameters were found (detailed equations not shown) predicting the opioid effects on tiredness and visual acuity with acceptable precision (RMSE 23.1 and 17.3 %, respectively), while multilinear predictions of nausea and drowsiness remained unacceptable (RMSE 32.6 and 26.5 %). The results did not substantially change with other permutations of outlier exclusions. Inclusion of the hyperalgesia case raised the RMSE of the prediction of analgesia by a linear combination of the three surrogate parameters to 31.6% which is inadequate. Finally, combined surrogates were also suitable for prediction of the blood concentrations (RMSE = 1.3 ng/ml; detailed equation see Figure 1).

To obtain a suitable prediction of analgesia, a more complex approach was required. From the present invention can be concluded that combined surrogates may provide better surrogates than single surrogates, without jeopardizing the prediction of blood concentrations. In the present setting, the improvement seems to be obtained by the combination of the pupil derived parameters, that highly correlate with remifentanil blood concentrations, with a parameter that covered the analgesic potency of remifentanil (reportedly ranging between 2 and 6 ng/ml (Kern and Stanski, 2008), presently provided by the tracking performance. Indeed, applying a sigmoid pharmacodynamic model to the effects on tracking performance resulted in a value of $EC_{50}$ of 5 ng/ml (standard error of estimate = 1, R$^2$ = 0.667). Nevertheless, this parameter may be replaced in other settings, provided that the newly chosen surrogate parameter meets the above criteria and is produced at a similar or lower potency as compared to that of analgesia.

**Claims**

1. A Method for the non-invasive prediction and/or monitoring of analgesia, and/or opioid blood-concentrations, in a subject who received at least one opioid dose, comprising acquiring at least one surrogate marker of said subject before, and at the same time or at least once after, the opioid administration; and determining therefrom said analgesia, or opioid blood-concentration, in said subject.

2. The method according to claim 1, wherein at least two, three or more surrogate markers are acquired of said patient, and wherein said analgesia, or opioid blood-concentration, is determined by linear combination of said two, three or more surrogate markers.

3. The method according to claim 1 or 2, wherein said surrogate marker is selected from the group consisting of pupil size, pupil reaction to light stimuli, tracking test performance, respiratory rate, arterial oxygenation, partial pressure of transcutaneous carbon dioxide, EEG derived parameters, clinical parameters of tiredness, nausea, drowsiness, vertigo, and itching.

4. The method according to any one of claims 1 to 3 wherein said blood opioid concentration is determined according to equation I:

$$\textbf{(I)} \qquad C = Effect \cdot EC_{50}/(E_{max} - Effect),$$

wherein
C is the predicted blood-concentration of said opioid,
*Effect* is the relative change of said surrogate marker from the baseline $EC_{50}$ is the concentration of said opioid at half maximum effect $E_{max}$ is the maximum effect.

5. The method according to any one of claims 1 to 4, wherein said opioid is remifentanil, optionally wherein said *Effect* is provided in percent change from baseline, and/or said $EC_{50}$ for remifentanil is 0.6 ng/ml, and/or said $E_{max}$ is -100%.

6. The method according to any one of claims 1 to 5, wherein the opioid blood-concentration is determined by equation (II)

$$\textbf{(II):} \quad C_{predicted} = k \cdot Effect_{\text{Pupil diameter}} - m \cdot Effect_{\text{Light reflex amplitude}} - n \cdot Effect_{\text{Tracking performance}} + w,$$

wherein k, m, n and w, are coefficients ranging from $-\infty$ to $\infty$, depending on the scaling.

7. The method according to claim 6, wherein k is 0.10316, m is 0.095613, n is 0.060719 and w is 0.038027.

8. The method according to any one of claims 1 to 5, wherein said analgesia is predicted by linear combination of all three surrogate markers according to equation (III)

$$\textbf{(III):} \quad Analgesia_{predicted} = q \cdot Effect_{\text{Pupil diameter}} - s \cdot Effect_{\text{Light reflex amplitude}} + t \cdot Effect_{\text{Tracking performance}} + v,$$

wherein
*Effect* is the relative change of said surrogate marker from the baseline (percent from baseline), and
q, s, t, v are coefficients ranging from $-\infty$ to $\infty$, depending on the scaling.

9. The method according to claim 8, wherein q is 4.7547, s is 2.4799, t is 0.45914 and v is 47.7756.

10. The method according to any one of claims 6 to 9, wherein said opioid is remifentanil.

11. An apparatus for performing the method of any one of claims 1 to 10, comprising

    a. Means for acquiring said at least one surrogate marker of a subject, and/or means for accepting as input said at least one surrogate marker,
b. Means for storing the recorded values of said at least one surrogate markers,
c. Means for calculating or monitoring said predicted analgesia, or opioid blood-concentrations, and
d. Optionally, means for display/output of the calculated analgesia, or opioid blood-concentrations.

12. The apparatus according to claim 11, further comprising means for applying a dose of said opioid to a subject, preferably wherein the amount and/or infusion rate of said dose of said opioid is calculated based on the predicted analgesia, and/or predicted opioid blood-concentration.

13. The apparatus according to claim 11 or 12, further comprising means for acquiring respiratory depression in said subject, and/or means for accepting as input the parameter for respiratory depression of said subject.

14. The apparatus according to any one of claims 11 to 13 for use in medicine.

15. The apparatus according to any one of claims 11 to 14 for use in the pain treatment of a patient, preferably a pain treatment involving the opioid remifentanil.

**Figure 1**

**Figure 2:**

Europäisches
Patentamt

European
Patent Office

Office européen
des brevets

## EUROPEAN SEARCH REPORT

Application Number

EP 13 16 5814

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/147505 A2 (UNIV BERN [CH]; ETH ZUERICH [CH]; BOUILLON THOMAS [CH]; CARUSO ANTONEL) 27 December 2007 (2007-12-27) | 1-6,8, 10-15 | INV. A61B5/11 A61B5/00 |
| A | * page 16, lines 4-18 * <br> * page 22, line 3 - page 25, line 3 * <br> * page 26, line 7 - page 30, line 10 * <br> * figures 1-2 * | 7,9 | |
| X | US 2005/039742 A1 (HICKLE RANDALL S [US]) 24 February 2005 (2005-02-24) | 1-6,8, 10-15 | |
| A | * paragraphs [0011] - [0012], [0017] - [0019], [0023], [0027] * <br> * figures 1, 4 * | 7,9 | |

TECHNICAL FIELDS SEARCHED (IPC)

A61B

The present search report has been drawn up for all claims

| Place of search | Date of completion of the search | Examiner |
|---|---|---|
| The Hague | 28 August 2013 | Faymann, Juan |

EPO FORM 1503 03.82 (P04C01)

**ANNEX TO THE EUROPEAN SEARCH REPORT**
**ON EUROPEAN PATENT APPLICATION NO.**

EP 13 16 5814

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2013

| Patent document cited in search report | | Publication date | Patent family member(s) | | Publication date |
|---|---|---|---|---|---|
| WO 2007147505 | A2 | 27-12-2007 | CN | 101472631 A | 01-07-2009 |
| | | | EP | 2029197 A2 | 04-03-2009 |
| | | | JP | 2009540890 A | 26-11-2009 |
| | | | WO | 2007147505 A2 | 27-12-2007 |
| US 2005039742 | A1 | 24-02-2005 | AU | 2003275460 A1 | 23-04-2004 |
| | | | AU | 2009213027 A1 | 08-10-2009 |
| | | | CA | 2507488 A1 | 15-04-2004 |
| | | | CN | 1720074 A | 11-01-2006 |
| | | | EP | 1554003 A2 | 20-07-2005 |
| | | | JP | 2006503617 A | 02-02-2006 |
| | | | US | 2005039742 A1 | 24-02-2005 |
| | | | WO | 2004030525 A2 | 15-04-2004 |

EPO FORM P0459

For more details about this annex : see Official Journal of the European Patent Office, No. 12/82